# EUROPEAN PATENT SPECIFICATION

(11) **EP 3 478 272 B1**
(45) Date of publication and mention of the grant of the patent: **18.11.2026**
(21) Application number: 17737028.5
(22) Date of filing: 30.06.2017
(51) Int. Cl.: A61K 9/48, A61K 31/352

(54) **CANNABINOID FORMULATIONS**
CANNABINOIDFORMULIERUNGEN
FORMULATIONS DE CANNABINOÏDES

(30) Priority: 01.07.2016 GB 201611544
(43) Date of publication of application: 08.05.2019
(73) Proprietor: Jazz Pharmaceuticals Research UK Limited, Sittingbourne Kent ME9 8AG (GB)
(72) Inventor: WILKHU, Jitinder, Sittingbourne, Kent ME9 8AG (GB); BENDER, Johan, 6572 AB Berg en Dal (NL)
(74) Representative: HGF
(86) International application number: PCT/GB2017/051943
(87) International publication number: WO 2018/002665

(56) References cited:
- US-A1- 2012 183 606
- DATABASE WPI Week 201381, Derwent World Patents Index; AN 2013-R00947, XP002773372
- DATABASE WPI Week 200821, Derwent World Patents Index; AN 2008-C80882, XP002773373
- THUMMA S ET AL: "Influence of plasticizers on the stability and release of a prodrug of @D^9-tetrahydrocannabinol incorporated in poly (ethylene oxide) matrices", EUROPEAN JOURNAL OF PHARMACEUTICS AND BIOPHARMACEUTICS, ELSEVIER SCIENCE PUBLISHERS B.V., AMSTERDAM, NL, vol. 70, no. 2, 1 October 2008 (2008-10-01), pages 605 - 614, XP025470907, ISSN: 0939-6411, [retrieved on 20080618], DOI: 10.1016/J.EJPB.2008.06.009
- EDWARD MAA ET AL: "The case for medical marijuana in epilepsy", EPILEPSIA, vol. 55, no. 6, 1 June 2014 (2014-06-01), pages 783 - 786, XP055205357, ISSN: 0013-9580, DOI: 10.1111/epi.12610

## Description

### Field of the Invention

The present invention relates to an oral pharmaceutical formulation comprising a cannabinoid. The formulation may take the form of a mucoadhesive gel, a tablet, a powder, a liquid gel capsule, an oral solution, granules, extrudates or injectable.

### Background of the Invention

Cannabinoids are lipophilic substances that are known to be poorly soluble in water (less than 1 µg/mL). As an example, CBD is soluble in ethanol (36 mg/mL) and dimethylsulfoxide DMSO (60 mg/mL).

Bioavailability of pharmaceutical substances taken perorally, first of all, depends on the extent to which the pharmaceutically active substance is absorbed from the intestinal environment across the intestinal mucosa. Lipophilic pharmaceutical substances are generally poorly absorbed from the intestinal environment, inter alia because of their poor solubility and/or dispersibility in water. Bioavailability of a pharmaceutical substance taken perorally furthermore depends on the susceptibility of the substance to the so-called first pass effect. Substances absorbed from the intestine, before being distributed throughout the body, have to pass the liver first where they may be metabolised immediately. CBD is generally assumed to be rather susceptible to first-pass liver metabolisation. Oral bioavailability of CBD is low and unpredictable (S. Zhornitsky, S. Potvin, Pharmaceuticals (2012) 5, 529-552). In addition, CBD is an unstable drug (A. J. Poortman, H. Huizer, Forensic Science International (1999) 101, 1-8).

In WO 2012/033478, Self Emulsifying Drug Delivery Systems (SEDDS) have been used to offer improved administration of cannabinoids.

SEDDS (self-emulsifying drug delivery systems) generally consist of hard or soft capsules filled with a liquid or a gel that consists of lipophilic active pharmaceutical ingredient (API), oil (to dissolve the API) and a surfactant. Upon contact with gastric fluid, the SEDDS spontaneously emulsify due to the presence of surfactants. Many surfactants, however, are lipid based and interact with lipases in the GIT. This can lead to a reduced capability of the lipid based surfactants to emulsify the API as well as the oil carrier, both reducing bioavailability.

In WO 2015/184127, an alcohol-free formulation comprising a cannabinoid, a polyethylene glycol and propylene glycol is disclosed.

CN 103110582 A discloses a pharmaceutical formulation comprising cannabidiol, a poloxamer 188 or 124, and propylene glycol. The formulation is a microemulsion comprising an oily phase, suitable for oral administration.

In WO 2012/033478, SEDDS formulations based on Type I, Type II and Type III were utilised.

The Lipid Formulation Classification System (LFCS) was introduced to help identify the characteristics of lipid systems (C.W. Pouton, Eur. J. Pharm. Sci., 11 (Suppl. 2) (2000), pp. S93-S98). As classified in the LFCS, Type I formulations are oils which require digestion, Type II formulations are water-insoluble self-emulsifying drug delivery systems (SEDDS), Type III systems are SEDDS or self-micro emulsifying drug delivery systems (SMEDDS) or self-nano emulsifying drug delivery systems (SNEDDS) which contain some water-soluble surfactants and/or co-solvents (Type IIIA) or a greater proportion of water soluble components (Type IIIB). Category Type IV represents a recent trend towards formulations which contain predominantly hydrophilic excipient surfactants and co-solvents. Below is a tabular Lipid Formulation Classification System overview taken from US 2015/111939:

| | Content of formulation (wt.-%) | | | | |
|---|---|---|---|---|---|
| Excipients in formulation | Type I | Type II | Type IIIA | Type IIIB | Type IV |
| Oil: triglycerides or mixed mono- and diglycerides | 100 | 40-80 | 40-80 | <20 | - |
| Water-insoluble surfactants (HLB < 12) | - | 20-60 | - | - | 0-20 |
| Water-soluble surfactants (HLB > 12) | - | - | 20-40 | 20-50 | 30-80 |
| Hydrophilic co-solvent | - | - | 0-40 | 20-50 | 0-50 |

A further description of the Lipid Formulation Classification System can also be found in *FABAD J. Pharm. Sci.,* pages 55-64, 2013.

As can be seen in the above table, Type IIIB formulations comprise <20 wt% of oil, based on the total composition. However, it should be noted that, by definition, Type IIIB formulations contain some oil, even if it is only a very small amount.

There is a need to provide an oral pharmaceutical formulation comprising a cannabinoid, wherein the formulation has improved drug-like properties such as bioavailability and stability.

### Brief Summary of the Invention

The present invention relates to a novel cannabinoid oral pharmaceutical dosage form as disclosed in claim 1, based on a Type IV or Type IV-like formulation, as classified using the Lipid Formulation Classification System. By Type IV-like, it is meant that the formulation comprises no oil, for example no triglycerides or mixed glycerides. When a Type IV-like formulation is used, it may comprise more than the 50 wt% of solvent, based on the total composition, as specified in the LFCS table.

In a first aspect of the invention, there is provided an oral pharmaceutical formulation comprising at least one cannabinoid in an amount of 10 to 50 wt%; at least one poloxamer which is defined according to formula (II) 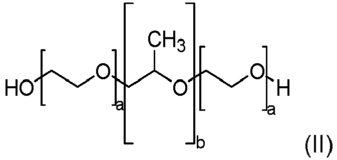
wherein each a is independently an integer of from 10 to 110 and b is an integer of from 20 to 60, in an amount of 30 to 60 wt%;
and a solvent, wherein the solvent is selected from diacetin, propylene glycol, triacetin, monoacetin, propylene glycol diacetate, triethyl citrate and mixtures thereof, in an amount of 20 to 50 wt%; and
wherein the formulation is substantially oil-free and wherein the oil is a triglyceride, a diglyceride, a monoglyceride, a fatty acid, a fatty acid ester or a mixture thereof; and
wherein the wt% are based on the total composition.

Also disclosed is an oral pharmaceutical dosage form or formulation comprises at least one cannabinoid; at least one poloxamer; and a solvent, wherein the solvent is defined according to formula (I) 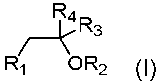 wherein R₁ and R₂ are independently selected from hydrogen, C(O)CH₃, OH, C(O)CH₃, CH₂OH and C(O)OCH₂CH₃; R₃ is independently selected from CH₃, CH₂OH, OH, CH₂OC(O)CH₃ and CH₂C(O)CH₂CH₃; and R₄ is independently selected from hydrogen and C(O)OCH₂CH₃.

This formulation enhances cannabinoid bioavailability compared to other formulations based on Type I, Type II, Type IIIA and Type IIIB, as classified by the Lipid Formulation Classification System. Accordingly, the oral pharmaceutical dosage form or formulation is not oil-based, i.e. it comprises substantially no oil. By "substantially no oil" or "substantially oil-free", it is meant that the formulation comprises less than 2 wt% oil, preferably less than 1 wt% based on the total composition. Such formulations are classified as Type IV or Type IV-like.

By enhancing bioavailability, the total amount of cannabinoid and excipients required during a certain window of time in a treatment of a specific disease may be reduced.

The formulation according to the present invention exhibits excellent stability under various storage conditions.

By enhancing stability, the length of time for which the formulations are fit for consumption may be increased.

### Detailed Description of the Invention

### The Cannabinoid

The formulation according to the present invention comprises at least one cannabinoid selected from the group consisting of cannabichromene (CBC), cannabichromenic acid (CBCV), cannabidiol (CBD), cannabidiolic acid (CBDA), cannabidivarin (CBDV), cannabigerol (CBG), cannabigerol propyl variant (CBGV), cannabicyclol (CBL), cannabinol (CBN), cannabinol propyl variant (CBNV), cannabitriol (CBO), tetrahydrocannabinol (THC), tetrahydrocannabinolic acid (THCA), tetrahydrocannabivarin (THCV) and tetrahydrocannabivarinic acid (THCVA). This list is not exhaustive and merely details the cannabinoids which are identified in the present application for reference. So far, over 100 different cannabinoids have been identified and these cannabinoids can be split into different groups as follows: Phytocannabinoids; Endocannabinoids; and Synthetic cannabinoids.

The formulation according to the present invention may also comprise at least one cannabinoid selected from those disclosed in Handbook of Cannabis, Roger Pertwee, Chapter 1, pages 3 to 15.

It is preferred that the formulation comprises only one or two cannabinoids, which are preferably selected from the group consisting of, cannabidiol (CBD) or cannabidivarin (CBDV), tetrahydrocannabivarin (THCV), cannabigerol (CBG) and cannabidiolic acid (CBDA) or a combination thereof. It is preferred that the formulation comprises cannabidiol and/or cannabidivarin.

The cannabinoid is present in an amount of from 10 to 50 wt%, based on the total composition. Preferably, the cannabinoid is present in an amount from about 20 to 30 wt%. The cannabinoid may be present in an amount of about 30 wt%.

Preferably, the cannabinoid is synthetic or highly purified from its natural source (for example, plant derived recrystallized form). When a highly purified source is used, it is purified such that the cannabinoid is present at greater than 95% of the total extract (w/w). Use of a synthetic or highly purified cannabinoid is advantageous because these contain relatively low amounts of wax. This assists in prevention of the formation of an oily formulation, increasing physical stability of the formulation.

The unit dose of cannabinoid in the oral pharmaceutical formulation may be in the range of from 0.001 to 350 mg, preferably 0.1 to 350 mg, more preferably 1 to 250 mg.

For example, it is envisaged that, when in tablet or capsule unit dose form, the amount of cannabinoid present may be 0.5, 2, 10, 25, 50, 100, 150, 200, 250, 300 or 350 mg.

The amount of cannabinoid present in the formulation may be 20 to 30 wt%, based on the total composition. It has been found that the formulation is stable and is a solid at room temperature and pressure (defined herein as 20 °C and 1 atm) even when the content of cannabinoid is relatively high, such as 25, 30 or 35 wt%. Without wishing to be bound by theory, it is believed that at least one poloxamer is essential to the stability of the formulation, particularly for high cannabinoid content.

### The Solvent

Also disclosed are formulations comprising a solvent, defined according to formula (I) 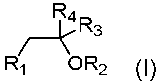 wherein R₁ and R₂ are independently selected from hydrogen, C(O)CH₃, OH, C(O)CH₃, CH₂OH and C(O)OCH₂CH₃; R₃ is independently selected from CH₃, CH₂OH, OH, CH₂OC(O)CH₃ and CH₂C(O)CH₂CH₃; and R₄ is independently selected from hydrogen and C(O)OCH₂CH₃.

According to the first aspect, the solvent is selected from the group consisting of diacetin, propylene glycol, triacetin, monoacetin, propylene glycol diacetate, triethyl citrate and mixtures thereof.

Diacetin is also known as glycerol diacetate.

Triacetin is also known as 1,2,3-triacetoxypropane, 1,2,3-triacetylglycerol or glycerol triacetate.

Monoacetin is also known as glycerol monoacetate or glycerol acetate.

Triethyl citrate is also known as citric acid ethyl ester.

Propylene glycol, propylene glycol diacetate and triethyl citrate are preferred solvents. Preferably, the solvent is triethyl citrate or propylene glycol.

The solvent is present in an amount of from 20 to 50 wt%, based on the total composition, preferably about 20 to 30 wt%. The solvent may be present in an amount of about 25 wt%.

When the solvent used is diacetin, it is preferred that it is present in an amount of from about 20 to 50 wt%, based on the total composition.

When the solvent used is propylene glycol, it is preferred that it is present in an amount of from about 20 to 30 wt%, based on the total composition.

When the solvent is triacetin, it is preferred that it is present in an amount of from about 20 to 50 wt%, based on the total composition.

When the solvent is triethyl citrate, it is preferred that it is present in an amount of from about 20 to 50 wt%, based on the total composition, more preferably about 20 to 30 wt%.

When the solvent is propylene glycol diacetate, it is preferred that it is present in an amount of from about 20 to 50 wt%, based on the total composition.

When only one poloxamer is present, as will be described below, it is preferred that the solvent is present in an amount of from about 45 to 55 wt%, preferably 45 to 50 wt%, based on the total composition.

The solvent or mixture of solvents according to the claimed invention may be the only solvent in the formulation. For example, the formulation may be substantially water-free and/or substantially alcohol-free, and the formulation is substantially oil-free. By "substantially water-free", "substantially alcohol-free" and "substantially oil-free", it is meant that the formulation comprises less than 2 wt%, preferably less than 1 wt% water, alcohol and/or oil based on the total composition.

The formulation is preferably substantially free from ethanol. More preferably the formulation is substantially alcohol-free.

In some embodiments the formulation is used in a paediatric patient, i.e. a patient under 18 years of age. In paediatric patients, it may be preferred that the formulation is substantially alcohol-free.

In a preferred embodiment, the formulation may be substantially free from or comprise no triglycerides, diglycerides or monoglycerides or mixtures thereof derived from glycerol and at least one fatty acid selected from the group consisting of caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid and docosahexaenoic acid and mixtures thereof. Preferably the formulation may be substantially free from or comprise no triglycerides, diglycerides or monoglycerides or mixtures thereof.

The formulation may be substantially free from hydrogenated vegetable oils, nut oils, anise oil, soybean oil, hydrogenated soybean oil, apricot kernel oil, corn oil, olive oil, peanut oil, almond oil, walnut oil, cashew oil, rice bran oil, poppy seed oil, cottonseed oil, canola oil, sesame oil, hydrogenated sesame oil, coconut oil, flaxseed oil, cinnamon oil, clove oil, nutmeg oil, coriander oil, lemon oil, orange oil, safflower oil, cocoa butter, palm oil, palm kernel oil, sunflower oil, rapeseed oil, castor oil, hydrogenated castor oil, polyoxyethylene castor oil derivatives, borage oil, beeswax, lanolin, petroleum jelly, mineral oil and light mineral oil.

More preferably the formulation may be free from triglycerides, diglycerides or monoglycerides or mixtures thereof derived from glycerol and caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid and docosahexaenoic acid and mixtures thereof, hydrogenated vegetable oils, nut oils, anise oil, soybean oil, hydrogenated soybean oil, apricot kernel oil, corn oil, olive oil, peanut oil, almond oil, walnut oil, cashew oil, rice bran oil, poppy seed oil, cottonseed oil, canola oil, sesame oil, hydrogenated sesame oil, coconut oil, flaxseed oil, cinnamon oil, clove oil, nutmeg oil, coriander oil, lemon oil, orange oil, safflower oil, cocoa butter, palm oil, palm kernel oil, sunflower oil, rapeseed oil, castor oil, hydrogenated castor oil, polyoxyethylene castor oil derivatives, borage oil, beeswax, lanolin, petroleum jelly, mineral oil and light mineral oil.

Even more preferably the formulation may be oil-free.

### The Poloxamer

The formulation according to the present invention comprises at least one poloxamer which is defined according to formula (II) 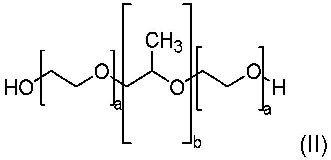 wherein a is an integer of from 10 to 110 and b is an integer of from 20 to 60.

It is preferred that when a is 12, b is 20. When a is 12 and b is 20, this is known as poloxamer 124.

It is also preferred that when a is 80, b is 27. When a is 80 and b is 27, this is known as poloxamer 188.

The formulation may comprise two poloxamers. When the formulation comprises two poloxamers, it is preferred that they are poloxamer 124 and poloxamer 188.

Other known poloxamers useful in the present invention are poloxamer 237 (a = 64; and b = 37), poloxamer 338 (a = 141; and b = 44) and poloxamer 407 (a = 101; and b = 56).

Further poloxamers that are known and can be useful in the present invention include poloxamer 108, poloxamer 182, poloxamer 183, poloxamer 212, poloxamer 217, poloxamer 238, poloxamer 288, poloxamer 331, poloxamer 338 and poloxamer 335.

The total amount of poloxamer present may be in an amount of from about 25 to 75 wt%, based on the total composition. Preferably the total amount of poloxamer present may be in the range of from about 25 to 60 wt% or 30 to 60 wt%, based on the total composition. More preferably the total amount of poloxamer present is from about 40 to about 50 wt%. The total amount of poloxamer present may be about 45 wt%.

When the formulation comprises poloxamer 124 and poloxamer 188, the amount of poloxamer 124 may be 5 wt% and the amount of poloxamer 188 may be 40 wt%, based on the total composition.

In some cases, the formulation may comprise only one poloxamer, wherein the poloxamer is poloxamer 188.

It has been found that, when poloxamer 407 is used, it is preferred that poloxamer 124 is present.

It has been found that the formulation of the invention has excellent rehydration properties. The formulation rehydrates rapidly and homogeneously. Upon rehydration the formulation has excellent release properties.

It has been found that the formulation of the invention has excellent stability. Without wishing to be bound by theory, it is believed that the presence of at least one poloxamer in the formulation affords excellent stability.

### Additional Agents

The formulation may further comprise an antioxidant, preferably in an amount of from about 0.001 to 5 wt%, more preferably about 0.001 to 2.5 wt%, based on the total composition.

The antioxidant may be selected from the group consisting of butylated hydroxytoluene, butylated hydroxyl anisole, alpha- tocopherol (Vitamin E), ascorbyl palmitate, ascorbic acid, sodium ascorbate, ethylenediamino tetraacetic acid, cysteine hydrochloride, citric acid, sodium citrate, sodium bisulfate, sodium metabisulfite, lecithin, propyl gallate, sodium sulfate, monothioglycerol and mixtures thereof.

A preferred group of antioxidants is alpha- tocopherol (Vitamin E), monothioglycerol, ascorbic acid, citric acid and mixtures thereof.

When the cannabinoid, CBDV, is present in the formulation, it is preferred that an antioxidant is also present.

The formulation may additionally comprise a flavouring agent, such as peppermint.

The formulation may additionally comprise a sweetener, such as sucrose.

### Preferred Formulations

It is preferred that the type IV oral formulation according to the invention is a solid at room temperature and pressure, i.e. preferably the formulation is a solid at 20 °C and 1 atm. Such formulations are typically fluid during manufacture, solid at room temperature and become fluid again at 37 °C. For the purposes of the invention, a gel is considered to be a solid.

Preferably the formulation comprises at least one cannabinoid, wherein the cannabinoid is CBD; at least two poloxamers, wherein the poloxamers are poloxamer 124 and poloxamer 188; and a solvent, wherein the solvent is triethyl citrate.

The disclosure also describes a formulation which consists of at least one cannabinoid; at least one poloxamer; a solvent; and optionally an antioxidant, wherein the solvent is defined according to formula (I) 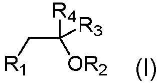 wherein R₁ and R₂ are independently selected from hydrogen, C(O)CH₃, OH, C(O)CH₃, CH₂OH and C(O)OCH₂CH₃; R₃ is independently selected from CH₃, CH₂OH, OH, CH₂OC(O)CH₃ and CH₂C(O)CH₂CH₃; and R₄ is independently selected from hydrogen and C(O)OCH₂CH₃.

The following represent preferred formulations according to the invention that are capable of forming a gel at body temperature.

A preferred oral pharmaceutical formulation (solid gel at room temperature) comprises
25 wt% cannabidiol;
34 wt% poloxamer 124;
15 wt% poloxamer 188; and
26 wt% propylene glycol.

A further preferred oral pharmaceutical formulation (Gel at room temperature) comprises
25 wt% cannabidiol;
34 wt% poloxamer 124;
15 wt% poloxamer 188; and
26 wt% diacetin.

A further preferred oral pharmaceutical formulation (Semi- solid gel at room temperature) comprises
25 wt% cannabidiol;
25 wt% poloxamer 124;
25 wt% poloxamer 407; and
25 wt% propylene glycol.

A further preferred oral pharmaceutical formulation (Solid at room temperature) comprises
25 wt% cannabidiol;
35 wt% poloxamer 124;
20 wt% poloxamer 188; and
20 wt% propylene glycol.

A further preferred oral pharmaceutical formulation (Gel at room temperature) comprises
35 wt% cannabidiol;
28 wt% poloxamer 124;
16 wt% poloxamer 188; and 22 wt% propylene glycol.

A further preferred oral pharmaceutical formulation (Solid at room temperature) comprises
12.5 wt% cannabidiol;
38 wt% poloxamer 124;
19 wt% poloxamer 188; and 30 wt% propylene glycol.

A further preferred oral pharmaceutical formulation (Gel at room temperature) comprises
25 wt% cannabidiol;
27 wt% poloxamer 188; and
48 wt% diacetin.

A further preferred oral pharmaceutical formulation (Gel at room temperature) comprises
30 wt% cannabidiol;
27 wt% poloxamer 188; and
43 wt% diacetin.

A further preferred oral pharmaceutical formulation (Gel at room temperature) comprises
25 wt% cannabidiol;
27 wt% poloxamer 188; and
48 wt% triacetin.

A further preferred oral pharmaceutical formulation (Semi-solid gel at room temperature) comprises
25 wt% cannabidiol;
27 wt% poloxamer 188; and
48 wt% propylene glycol.

A further preferred oral pharmaceutical formulation (Solid at room temperature) comprises
25 wt% cannabidiol;
35 wt% poloxamer 124;
20 wt% poloxamer 188; and
20 wt% triethyl citrate.

A further preferred oral pharmaceutical formulation (Gel at room temperature) comprises
25 wt% cannabidiol;
27 wt% poloxamer 188; and
48 wt% triethyl citrate.

A further preferred oral pharmaceutical formulation (Gel at room temperature) comprises
25 wt% cannabidivarin;
27 wt% poloxamer 188; and
48 wt% triethyl citrate.

A further preferred oral pharmaceutical formulation (Solid at room temperature) comprises
25 wt% cannabidivarin;
35 wt% poloxamer 124;
20 wt% poloxamer 188; and
20 wt% propylene glycol.

A further preferred oral pharmaceutical formulation (Solid at room temperature) comprises
20 wt% cannabidivarin;
35 wt% poloxamer 124;
25 wt% poloxamer 188; and
20 wt% triacetin.

A further preferred oral pharmaceutical formulation (Solid at room temperature) comprises
25 wt% cannabidivarin;
35 wt% poloxamer 124;
20 wt% poloxamer 188; and
20 wt% triethyl citrate.

### Treatment

The formulation is for use in therapy, preferably for use in paediatric epilepsy.

The formulation may also be used in the treatment of a disease or disorder selected from the group consisting of Dravet Syndrome, Lennox Gastaut Syndrome, myocolonic seizures, juvenile myocolonic epilepsy, refractory epilepsy, schizophrenia, juvenile spasms, West syndrome, infantile spasms, refractory infantile spasms, tuberous sclerosis complex, brain tumors, neuropathic pain, cannabis use disorder, post-traumatic stress disorder, anxiety, early psychosis, Alzheimer's Disease, and autism.

As already stated, cannabidiol is preferred for use in the present invention. Cannabidiol can be used in the treatment of atonic, absence or partial seizures, in particular, simple or complex seizures. It is particularly effective in reducing seizures in patients suffering with etiologies that include: Lennox-Gastaut Syndrome; Tuberous Sclerosis Complex; Dravet Syndrome; Doose Syndrome; CDKL5; Dup15q; Jeavons syndrome; Myoclonic Absence Epilepsy; Neuronal ceroid lipofuscinoses (NCL) and brain abnormalities.

In addition, a formulation comprising CBDV and/or CBDA can be used in the treatment of autism spectrum disorders, in particular Rett syndrome, Fragile X syndrome, Angelman syndrome, ADHD and hyperkinetic disorders, such as Tourette syndrome and dystonias. Thus, the formulation comprising CBDV and/or CBDA can be useful in a method of treatment of such disorders.

The formulation of the invention may be useful in a method of treating a patient having a disorder selected from the group consisting of Dravet Syndrome, Lennox Gastaut Syndrome, myoclonic seizures, juvenile myoclonic epilepsy, refractory epilepsy, schizophrenia, juvenile spasms, West syndrome, infantile spasms, refractory infantile spasms, tuberous sclerosis complex, brain tumors, neuropathic pain, cannabis use disorder, post-traumatic stress disorder, anxiety, early psychosis, Alzheimer's Disease, and autism.

When cannabidiol is used in the formulation, the formulation may be useful in a method of treatment of atonic, absence or partial seizures in a patient, in particular, simple or complex seizures. It is particularly effective in a method of reducing seizures in patients suffering with etiologies that include: Lennox-Gastaut Syndrome; Tuberous Sclerosis Complex; Dravet Syndrome; Doose Syndrome; CDKL5; Dup15q; Jeavons syndrome; Myoclonic Absence Epilepsy; Neuronal ceroid lipofuscinoses (NCL) and brain abnormalities.

The method of treatments comprise administering a patient with a therapeutically effective amount of a formulation or of a cannabinoid in a formulation according to the present invention.

### Definitions

"Cannabinoids" are a group of compounds including the endocannabinoids, the phytocannabinoids and those which are neither endocannabinoids or phytocannabinoids, hereinafter "syntho-cannabinoids".

"Endocannabinoids" are endogenous cannabinoids, which are high affinity ligands of CB1 and CB2 receptors.

"Phytocannabinoids" are cannabinoids that originate in nature and can be found in the cannabis plant. The phytocannabinoids can be present in an extract including a botanical drug substance, isolated, or reproduced synthetically.

"Syntho-cannabinoids" are those compounds capable of interacting with the cannabinoid receptors (CB1 and/or CB2) but are not found endogenously or in the cannabis plant. Examples include WIN 55212 and rimonabant.

An "isolated phytocannabinoid" is one which has been extracted from the cannabis plant and purified to such an extent that all the additional components such as secondary and minor cannabinoids and the non-cannabinoid fraction have been removed.

A "synthetic cannabinoid" is one which has been produced by chemical synthesis. This term includes modifying an isolated phytocannabinoid, by, for example, forming a pharmaceutically acceptable salt thereof.

A "substantially pure" cannabinoid is defined as a cannabinoid which is present at greater than 95% (w/w) pure. More preferably greater than 96% (w/w) through 97% (w/w) thorough 98% (w/w) to 99% % (w/w) and greater.

A "highly purified" cannabinoid is defined as a cannabinoid that has been extracted from the cannabis plant and purified to the extent that other cannabinoids and non-cannabinoid components that are co-extracted with the cannabinoids have been substantially removed, such that the highly purified cannabinoid is greater than or equal to 95% (w/w) pure.

A "botanical drug substance" or "BDS" is defined in the Guidance for Industry Botanical Drug Products Draft Guidance, August 2000, US Department of Health and Human Services, Food and Drug Administration Centre for Drug Evaluation and Research as: "A drug derived from one or more plants, algae, or microscopic fungi. It is prepared from botanical raw materials by one or more of the following processes: pulverisation, decoction, expression, aqueous extraction, ethanolic extraction or other similar processes."

A botanical drug substance does not include a highly purified or chemically modified substance derived from natural sources. Thus, in the case of cannabis, BDS derived from cannabis plants do not include highly purified Pharmacopoeial grade cannabinoids.

An "oil" is typically defined as a single compound or a mixture of compounds that are both hydrophobic and lipophilic. Exemplary oils include triglycerides, diglycerides, monoglycerides, fatty acids and fatty acid esters. Triglycerides, diglycerides and monoglycerides are esters derived from glycerol and three, two or one fatty acids. Diglycerides and triglycerides may have the same or they may have different fatty acids for each ester bond. Exemplary fatty acids include carboxylic acids with a saturated or unsaturated, linear or branched carbon chains, such as caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid and docosahexaenoic acid. Exemplary mixtures of oils include plant and animal fats and waxes such as vegetable oils, hydrogenated vegetable oils, nut oils, anise oil, soybean oil, hydrogenated soybean oil, apricot kernel oil, corn oil, olive oil, peanut oil, almond oil, walnut oil, cashew oil, rice bran oil, poppy seed oil, cottonseed oil, canola oil, sesame oil, hydrogenated sesame oil, coconut oil, flaxseed oil, cinnamon oil, clove oil, nutmeg oil, coriander oil, lemon oil, orange oil, safflower oil, cocoa butter, palm oil, palm kernel oil, sunflower oil, rapeseed oil, castor oil, hydrogenated castor oil, polyoxyethylene castor oil derivatives, borage oil, beeswax, lanolin, petroleum jelly, mineral oil and light mineral oil. For the purposes of the present invention cannabinoids are not considered to be oils.

An "alcohol" has its standard meaning within the art. It includes ethanol, propanol etc.

"Room temperature and pressure" is defined herein as 20 °C and 1 atm.

### Examples

Analytical procedures, cannabinoids and excipients used in the examples:

### 1. Rehydration (RH) procedure

A type IV oral pharmaceutical formulation (OPF) comprising at least one cannabinoid, at least one solvent and at least one poloxamer was rehydrated by adding 20 mL water for injections at room temperature (RH-RT) or by adding 20 mL water for injections at 37 °C (RH-37) in Class-3 glass colourless transparent vials. The vials were vortexed for 10 seconds.

### 2. Test for appearances

Appearance of OPF: the viscosity, homogeneity and clarity of the OPF was checked visually.

Appearance of rehydrated OPF: after rehydration, the formulation is checked visually on homogeneity and presence of particles and/or non-rehydrated OPF. The presence of foam is an indication that enough poloxamer is used to rehydrate the cannabinoid(s).

### 3. Release of cannabinoid in rehydration fluid

The release of cannabinoid in the rehydration fluid was tested as follows:
Rehydrated OPF was submitted for HPLC analysis. Equipment: HPLC system with variable wavelength UV detector or diode array detector. Column: Ace C18-AR 150 x 4.6 mm , 3 µm. Pre-Column: Ace C18-AR Guard Cartridge. Mobile Phase: Acetonitrile: 0.25% acetic acid (62%: 38%). Column Temperature: 38°C. Flow Rate: 1.0 ml min-1. Detection: 220 nm. Injection Volume: 10 µl. Run Time 25 minutes. Sample preparation: accurately prepare test samples at an approximate concentration of 0.15 mg/ml in triplicate. Samples may be prepared at a higher concentration to ensure accurate quantification of related substances or degradants. 0.1 mL rehydrated OPF was diluted with 10 mL ethanol; 10 µL was injected into the HPLC system.

### 4. Stability and stability tests

Stability of OPF was executed according to ICH Guidance Q1A - Q1F. Samples were stored at 25°C ± 2°C/60% RH ± 5%, 30°C ± 2°C/65% RH ± 5% RH and 40°C ± 2°C/75% RH ± 5%. Stability of OPF was assessed by chemical analysis and appearance. Chemical analysis was performed by a stability indicating HPLC method, described in [3]. The number of repeat experiments for each time point was 3, except at 6 months, when 6 repeat experiments were conducted. Sample preparation: 0.1 mL rehydrated OPF was diluted with 10 mL ethanol; 10 µL was injected into the HPLC system.

The following formulation was prepared for use in the stability study.

Type IV formulation (150 mg/capsule): 30% w/w CBD; 5% w/w P124; 40% w/w P188; and 25% w/w triethyl citrate.

### 5. Cannabinoids

CBD: synthetic, plant derived CBD containing waxes and plant derived recrystallized CBD (CBD-r). Plant derived CBDV and synthetic CBDV.

### 6. Excipients

Lutrol L44 (BASF, poloxamer 124: P124), Lutrol F68 (BASF, poloxamer 188: P188), Lutrol F87 (BASF, poloxamer 237: P237), Lutrol F108 (BASF, poloxamer 338: P338), Lutrol F127 (BASF, poloxamer 407, P407), glycerol (Sigma: gly), diacetin (Sigma: di), triacetin (Sigma: tri), propylene glycol (Sigma: PG), ethanol (Fischer), propylene glycol diacetate (Sigma: PGDA), triethyl citrate (Sigma: TEC).

### 7. Melt Procedure

The excipients and cannabinoids are weighed into a vessel and are heated until molten. Upon cooling the gel is filled into capsules or vials by weight. The viscosity of the gel is a function of temperature which enables the flexibility of filling into HPMC, Gelatin and soft-Gelatin capsules.

Alternatively, gel based formulations can be manufactured where the excipients and cannabinoids can be dissolved into an organic solvent such as, ethanol, methanol, propanol and filled into glass vials with a process step of evaporating the organic solvent off to leave the gel in the vial.

### Stability Study

The purpose of stability testing is to provide evidence on how the quality of a drug product varies with time under the influence of a variety of environmental factors such as temperature and humidity. In order to illustrate that the Type IV formulations according to the invention exhibit excellent stability, stability of OPF was executed according to ICH Guidance Q1A - Q1F.

The results of the stability study are represented in Tables 1-3 below. Table 1 presents the data for samples stored at 25°C ± 2°C/60% RH ± 5%. Table 2 presents the data for samples stored at 30°C ± 2°C/65% RH ± 5% RH. Table 3 presents the data for samples stored at 40°C ± 2°C/75% RH ± 5%.

**Table 1**

| | **Time Point (Months)** | | | |
|---|---|---|---|---|
| | **0** | **3** | **6** | **7** |
| **CBD Content (mg/Capsule)** | 149.13 | 149.56 | 149.54 | 147.70 |
| (% **of Initial CBD Content)** | 100.00 | 100.3 | 100.3 | 99.0 |

**Table 2**

| | **Time Point (Months)** | | | |
|---|---|---|---|---|
| | 0 | 3 | 6 | 7 |
| **CBD Content (mg/Capsule)** | 149.13 | 150.12 | 148.58 | 147.05 |
| (% **of Initial CBD Content)** | 100.00 | 100.7 | 99.6 | 98.6 |

**Table 3**

| | **Time Point (Months)** | | |
|---|---|---|---|
| | 0 | 3 | 6 |
| **CBD Content (mg/Capsule)** | 149.13 | 148.02 | 146.20 |
| (% **of Initial CBD Content)** | 100.00 | 99.3 | 98.0 |

As shown in Tables 1-3, the Type IV formulations according to the invention exhibit excellent stability, even under strenuous conditions, such as 40°C ± 2°C/75% RH ± 5%. Even under storage conditions of 40°C ± 2°C/75% RH ± 5%, 98% of the initial CBD content was recovered after 6 months.

In summary, it has been shown that a Type IV formulation according to the invention, exhibits excellent stability.

### Bioavailability Study

In order to illustrate that the Type IV formulations according to the invention exhibit improved bioavailability relative to Type I and Type III formulations, a comparison was made and bioavailability for each formulation measured. The results of the bioavailability study are represented in Table 4 below.

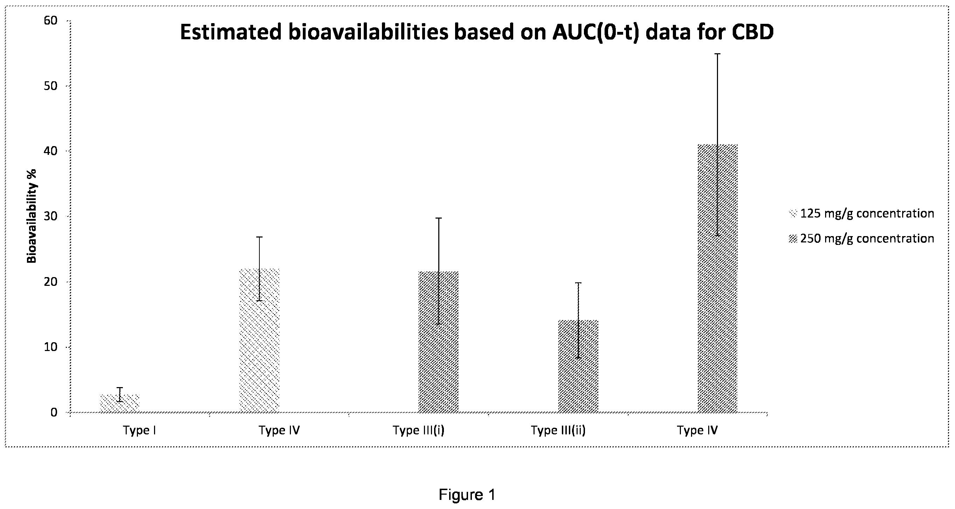

The outcome of the study is also depicted in Figure 1. As can be seen, the Type IV formulation, according to the present invention exhibits improved bioavailability compared to Type I and Type III formulations having the same concentration of CBD. As shown in Table 4, the result of subject 50 appears to be an anomaly because it falls outside of the general trend of improved bioavailability. This is clearly shown in Figure 1, despite inclusion of the anomaly.

In summary, it has been shown that a Type IV formulation, as classified by the Lipid Formulation Classification System, exhibits improved bioavailability for CBD.

### Details of the PK study for measurement of bioavailability

Beagle dogs (supplied by Charles River UK) received oral capsule doses at a target level of 15 mg/kg. Capsules used were size '0' gelatine capsules and the animals received a 100 mL water flush after each capsule was administered. The volume of blood taken at each sampling time was 2 mL and were collected mostly from the jugular vein. On a few occasions, cephalic vein samples were collected. The sampling times were: 0.5, 1, 1.5, 2, 2.5, 3, 4, 5, 6, 8, 12 and 24 h post-dose. The determination of CBD, 6-OH CBD, THC and 11 OH THC in dog plasma was performed by protein precipitation with reverse phase liquid chromatography with tandem mass spectrometric detection. The LLOQ of CBD was 1 ng/ml and all metabolites had an LLOQ of 0.5 ng/ml.

The human equivalent dose (HED) can be estimated using the following formula:
HED = Animal dose (mg/kg) *multiplied by Animal* Kₘ
Human Kₘ

The Kₘ for a dog is 20 and the Kₘ for a human is 37.

Thus, for a human a 15 mg/kg dose in a dog equates to a human dose of about 8.1 mg/kg.

### Formulations for measurement of bioavailability

Diacetin was weighed by weight into a vial followed by P124 directly on top. The P188 was weighed and added to the vessel containing the diacetin and P124. Finally, the desired amount of CBD is weighed and added to the vessel and heated (100 °C) until molten with a vortex to ensure a homogenous gel. Upon cooling (30-40 °C) the gel is filled into capsules or vials by weight. The viscosity of the gel is a function of temperature which enables the flexibility of filling into HPMC, Gelatin and soft-Gelatin capsules. At room temperature, low CBD dose gels were solid whereas the higher loaded CBD formulations remained a gel.

The following formulations were prepared for use in the PK study.

Type IV Gel (125 mg/g): 12.5% w/w CBD; 38% w/w P124; 19% w/w P188; and 30% w/w diacetin. Release = 99.3%. Appearance = solid gel.

Type IV Gel (250 mg/g): 25% w/w CBD; 34% w/w P124; 15% w/w P188; and 26% w/w diacetin. Release = 97.4%. Appearance = clear gel.

In both gel formulations, the CBD used was a highly purified form.

Type III(i) SEDDS (250 mg/g): CBD formulated with 15 wt% oil, 45 wt% water soluble surfactants and 40 wt% hydrophilic cosolvent.

Type III(ii) SEDDS (250 mg/g): CBD formulated with 31 wt% oil, 45 wt% water soluble surfactants and 24 wt% hydrophilic cosolvent.

**Table 4**

| **Estimated bioavailabilities based on AUC(0-t) data for CBD** | | | | | | | | | | | | | | | |
|---|---|---|---|---|---|---|---|---|---|---|---|---|---|---|---|
| | | **Subject** | | | | | | | | | | | | | |
| | | **47** | **48** | **49** | **50** | **57** | **58** | **59** | **60** | **61** | **62** | **63** | **N** | **Mean** | **SD** |
| **Analyte** | **Oral Formulation** | **Bioavailability_using_AUCt_for_CBD** | | | | | | | | | | | | | |
| Type I | Control Oil based (125 mg/g) | 4.43 | 2.95 | | | | 2.11 | 1.67 | 2.43 | | | | 5 | 2.72 | 1.07 |
| Type III(i) | SEDDS (250 mg/g) | 19.9 | 46.7 | | | | 15.5 | 20.0 | 27.0 | | | | 5 | 25.8 | 12.4 |
| Type III(ii) | SEDDS (250 mg/g) | | | 9.00 | 11.7 | 14.6 | | | | 6.62 | 6.65 | 16.3 | 6 | 10.8 | 4.09 |
| Type IV | Gel (125 mg/g) | | | 20.4 | | 31.1 | | | | 10.3 | 25.9 | 22.3 | 5 | 22.0 | 7.70 |
| Type IV | Gel (250 mg/g) | | | 37.2 | 17.3 | 38.0 | | | | 55.7 | 53.5 | 44.3 | 6 | 41.0 | 13.9 |

## Claims

1. An oral pharmaceutical formulation comprising at least one cannabinoid in an amount of 10 to 50 wt%;
at least one poloxamer which is defined according to formula (II) 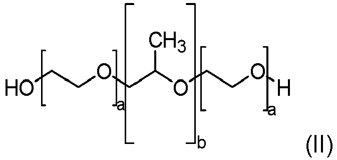
wherein each a is independently an integer of from 10 to 110 and b is an integer of from 20 to 60, in an amount of 30 to 60 wt%;
and a solvent, wherein the solvent is selected from diacetin, propylene glycol, triacetin, monoacetin, propylene glycol diacetate, triethyl citrate and mixtures thereof, in an amount of 20 to 50 wt%; and
wherein the formulation is substantially oil-free and wherein the oil is a triglyceride, a diglyceride, a monoglyceride, a fatty acid, a fatty acid ester or a mixture thereof; and
wherein the wt% are based on the total composition.

2. The formulation according to claim 1, wherein in formula (I) each a is 12 and b is 20.

3. The formulation according to claim 1, wherein in formula (I) each a is 80 and b is 27.

4. The formulation according to claim 1, wherein the poloxamer is poloxamer 124 or poloxamer 188, or a mixture thereof.

5. The formulation according to any one of the preceding claims, wherein the solvent is triethyl citrate.

6. The formulation according to any one of the preceding claims, wherein the cannabinoid is selected from the group consisting of cannabichromene (CBC), cannabichromenic acid (CBCV), cannabidiol (CBD), cannabidiolic acid (CBDA), cannabidivarin (CBDV), cannabigerol (CBG), cannabigerol propyl variant (CBGV), cannabicyclol (CBL), cannabinol (CBN), cannabinol propyl variant (CBNV), cannabitriol (CBO), tetrahydrocannabinol (THC), tetrahydrocannabinolic acid (THCA), tetrahydrocannabivarin (THCV) and tetrahydrocannabivarinic acid (THCVA) and combinations thereof.

7. The formulation according to any one of the preceding claims, wherein the cannabinoid is cannabidiol (CBD).

8. The formulation according to any one of the preceding claims, wherein the cannabinoid is synthetic or highly purified from its natural source.

9. The formulation according to any one of the preceding claims, wherein the cannabinoid is present in an amount of from 10 to 30 wt%, based on the total composition.

10. The formulation according to any one of the preceding claims, further comprising an antioxidant, in an amount of from 0.001 to 5 wt%, based on the total composition.

11. The formulation according to claim 10, wherein the antioxidant is selected from the group consisting of butylated hydroxyltoluene, butylated hydroxyl anisole, alphatocopherol (Vitamin E), ascorbyl palmitate, ascorbic acid, sodium ascorbate, ethylenediamino tetraacetic acid, cysteine hydrochloride, citric acid, sodium citrate, sodium bisulfate, sodium metabisulfite, lecithin, propyl gallate, sodium sulfate, monothioglycerol and mixtures thereof.

12. The formulation according to any one of the preceding claims, wherein the formulation is a solid at 20 °C and 1 atm.

13. The formulation according to any one of the preceding claims, wherein the formulation is an oral dosage form selected from the group consisting of mucoadhesive gel, a tablet, a powder, a liquid gel capsule, solid capsule, an oral solution, granule or extrudates.

14. The formulation according to any one of the preceding claims, for use in therapy.

15. The formulation according to any one of the preceding claims, for use in the treatment of a disease or disorder selected from the group consisting of Dravet Syndrome, Lennox Gastaut Syndrome, myoclonic seizures, juvenile myoclonic epilepsy, refractory epilepsy, schizophrenia, juvenile spasms, West syndrome, infantile spasms, refractory infantile spasms, tuberous sclerosis complex, brain tumors, neuropathic pain, cannabis use disorder, post-traumatic stress disorder, anxiety, early psychosis, Alzheimer's Disease, and autism.

## Patentansprüche

1. Orale pharmazeutische Formulierung, umfassend mindestens ein Cannabinoid in einer Menge von 10 bis 50 Gew.-%;
mindestens ein Poloxamer, das definiert ist gemäß Formel (II) 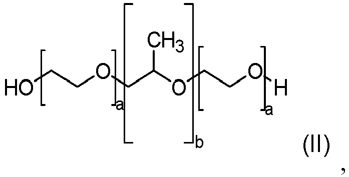
wobei jedes a unabhängig eine ganze Zahl von 10 bis 110 ist und b eine ganze Zahl von 20 bis 60 ist, in einer Menge von 30 bis 60 Gew.-%;
und ein Lösungsmittel, wobei das Lösungsmittel ausgewählt ist aus Diacetin, Propylenglycol, Triacetin, Monoacetin, Propylenglycoldiacetat, Triethylcitrat und Mischungen davon, in einer Menge von 20 bis 50 Gew.-%; und
wobei die Formulierung im Wesentlichen ölfrei ist und wobei das Öl ein Triglycerid, ein Diglycerid, ein Monoglycerid, eine Fettsäure, ein Fettsäureester oder eine Mischung davon ist; und
wobei die Gew.-% auf der Gesamtzusammensetzung basieren.

2. Formulierung nach Anspruch 1, wobei in Formel (I) jedes a 12 ist und b 20 ist.

3. Formulierung nach Anspruch 1, wobei in Formel (I) jedes a 80 ist und b 27 ist.

4. Formulierung nach Anspruch 1, wobei das Poloxamer Poloxamer 124 oder Poloxamer 188 oder eine Mischung davon ist.

5. Formulierung nach einem der vorhergehenden Ansprüche, wobei das Lösungsmittel Triethylcitrat ist.

6. Formulierung nach einem der vorhergehenden Ansprüche, wobei das Cannabinoid ausgewählt ist aus der Gruppe bestehend aus Cannabichromen (CBC), Cannabichromensäure (CBCV), Cannabidiol (CBD), Cannabidiolsäure (CBDA), Cannabidivarin (CBDV), Cannabigerol (CBG), Cannabigerolpropylvariante (CBGV), Cannabicyclol (CBL), Cannabinol (CBN), Cannabinolpropylvariante (CBNV), Cannabitriol (CBO), Tetrahydrocannabinol (THC), Tetrahydrocannabinolsäure (THCA), Tetrahydrocannabivarin (THCV) und Tetrahydrocannabivarinsäure (THCVA) und Kombinationen davon.

7. Formulierung nach einem der vorhergehenden Ansprüche, wobei das Cannabinoid Cannabidiol (CBD) ist.

8. Formulierung nach einem der vorhergehenden Ansprüche, wobei das Cannabinoid synthetisch oder hochgereinigt aus seiner natürlichen Quelle ist.

9. Formulierung nach einem der vorhergehenden Ansprüche, wobei das Cannabinoid in einer Menge von 10 bis 30 Gew.-%, basierend auf der Gesamtzusammensetzung, vorhanden ist.

10. Formulierung nach einem der vorhergehenden Ansprüche, ferner umfassend ein Antioxidans in einer Menge von 0,001 bis 5 Gew.-%, basierend auf der Gesamtzusammensetzung.

11. Formulierung nach Anspruch 10, wobei das Antioxidans ausgewählt ist aus der Gruppe bestehend aus butyliertem Hydroxyltoluol, butyliertem Hydroxylanisol, Alphatocopherol (Vitamin E), Ascorbylpalmitat, Ascorbinsäure, Natriumascorbat, Ethylendiamintetraessigsäure, Cysteinhydrochlorid, Zitronensäure, Natriumcitrat, Natriumbisulfat, Natriummetabisulfit, Lecithin, Propylgallat, Natriumsulfat, Monothioglycerin und Mischungen davon.

12. Formulierung nach einem der vorhergehenden Ansprüche, wobei die Formulierung bei 20 °C und 1 atm ein Feststoff ist.

13. Formulierung nach einem der vorhergehenden Ansprüche, wobei die Formulierung eine orale Darreichungsform ist, die aus der Gruppe ausgewählt ist, die aus mucoadhäsivem Gel, einer Tablette, einem Pulver, einer flüssigen Gelkapsel, festen Kapsel, einer oralen Lösung, Granulat oder Extrudaten besteht.

14. Formulierung nach einem der vorhergehenden Ansprüche zur Verwendung bei Therapie.

15. Formulierung nach einem der vorhergehenden Ansprüche zur Verwendung bei der Behandlung einer Krankheit oder Störung, die ausgewählt ist aus der Gruppe bestehend aus Dravet-Syndrom, Lennox-Gastaut-Syndrom, myoklonischen Anfällen, juveniler myoklonischer Epilepsie, refraktärer Epilepsie, Schizophrenie, juvenilen Spasmen, West-Syndrom, infantilen Spasmen, refraktären infantilen Spasmen, tuberösem Sklerosekomplex, Hirntumoren, neuropathischen Schmerzen, Cannabiskonsumstörung, posttraumatischer Belastungsstörung, Angst, früher Psychose, Alzheimer-Krankheit und Autismus.

## Revendications

1. Formulation pharmaceutique orale comprenant au moins un cannabinoïde en une quantité de 10 à 50 % en poids ;
au moins un poloxamère qui est défini selon la formule (II) 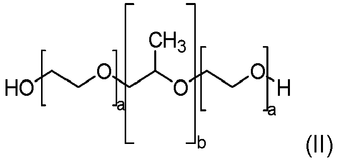
dans laquelle chaque a est indépendamment un nombre entier de 10 à 110 et b est un nombre entier de 20 à 60, en une quantité de 30 à 60 % en poids ;
et un solvant, ledit solvant étant choisi parmi la diacétine, le propylène glycol, la triacétine, la monoacétine, le diacétate de propylène glycol, le citrate de triéthyle et des mélanges de ceux-ci, en une quantité de 20 à 50 % en poids ; et
ladite formulation étant sensiblement exempte d'huile et la dite huile étant un triglycéride, un diglycéride, un monoglycéride, un acide gras, un ester d'acide gras ou un mélange de ceux-ci ; et
lesdits % en poids étant basés sur la composition totale.

2. Formulation selon la revendication 1, dans laquelle dans la formule (I), chaque a est 12 et b est 20.

3. Formulation selon la revendication 1, dans laquelle dans la formule (I), chaque a est 80 et b est 27.

4. Formulation selon la revendication 1, dans laquelle le poloxamère est le poloxamère 124 ou le poloxamère 188, ou un mélange de ceux-ci.

5. Formulation selon l'une quelconque des revendications précédentes, dans laquelle le solvant est le citrate de triéthyle.

6. Formulation selon l'une quelconque des revendications précédentes, dans laquelle le cannabinoïde est choisi dans le groupe constitué du cannabichromène (CBC), de l'acide cannabichroménique (CBCV), du cannabidiol (CBD), de l'acide cannabidiolique (CBDA), de la cannabidivarine (CBDV), du cannabigérol (CBG), d'une variante cannabigérol propyle (CBGV), du cannabicyclol (CBL), du cannabinol (CBN), d'une variante cannabinol propyle (CBNV), du cannabitriol (CBO), du tétrahydrocannabinol (THC), de l'acide tétrahydrocannabinolique (THCA), de la tétrahydrocannabivarine (THCV) et de l'acide tétrahydrocannabivarinique (THCVA) et des combinaisons de ceux-ci.

7. Formulation selon l'une quelconque des revendications précédentes, dans laquelle le cannabinoïde est le cannabidiol (CBD).

8. Formulation selon l'une quelconque des revendications précédentes, dans laquelle le cannabinoïde est synthétique ou hautement purifié à partir de sa source naturelle.

9. Formulation selon l'une quelconque des revendications précédentes, dans laquelle le cannabinoïde est présent en une quantité de 10 à 30 % en poids, sur la base de la composition totale.

10. Formulation selon l'une quelconque des revendications précédentes, comprenant en outre un antioxydant, en une quantité de 0,001 à 5 % en poids, sur la base de la composition totale.

11. Formulation selon la revendication 10, dans laquelle l'antioxydant est choisi dans le groupe constitué de l'hydroxyltoluène butylé, de l'hydroxylanisole butylé, de l'alphatocophérol (vitamine E), du palmitate d'ascorbyle, de l'acide ascorbique, de l'ascorbate de sodium, de l'acide éthylènediaminotétraacétique, du chlorhydrate de cystéine, de l'acide citrique, du citrate de sodium, du bisulfate de sodium, du métabisulfite de sodium, de la lécithine, du gallate de propyle, du sulfate de sodium, du monothioglycérol et des mélanges de ceux-ci.

12. Formulation selon l'une quelconque des revendications précédentes, ladite formulation étant un solide à 20 °C et 1 atm.

13. Formulation selon l'une quelconque des revendications précédentes, ladite formulation étant une forme posologique orale choisie dans le groupe constitué d'un gel mucoadhésif, d'un comprimé, d'une poudre, d'une capsule de gel liquide, d'une capsule solide, d'une solution orale, d'un granulé ou d'extrudés.

14. Formulation selon l'une quelconque des revendications précédentes, destinée à être utilisée en thérapie.

15. Formulation selon l'une quelconque des revendications précédentes, destinée à être utilisée dans le traitement d'une maladie ou d'un trouble choisi(e) dans le groupe constitué du syndrome de Dravet, du syndrome de Lennox Gastaut, des crises myocloniques, de l'épilepsie myoclonique juvénile, de l'épilepsie réfractaire, de la schizophrénie, des spasmes juvéniles, du syndrome de West, des spasmes infantiles, des spasmes infantiles réfractaires, de la sclérose tubéreuse complexe, des tumeurs cérébrales, d'une douleur neuropathique, d'un trouble lié à la consommation de cannabis, d'un trouble de stress post-traumatique, de l'anxiété, de la psychose précoce, de la maladie d'Alzheimer et de l'autisme.
